(19) Europäisches Patentamt
European Patent Office
Office européen des brevets

(11) EP 4 245 795 A1

(12) EUROPEAN PATENT APPLICATION
published in accordance with Art. 153(4) EPC

(43) Date of publication:
20.09.2023 Bulletin 2023/38

(21) Application number: 21903247.1

(22) Date of filing: 30.11.2021

(51) International Patent Classification (IPC):
C08J 3/12 (2006.01)       C08L 23/00 (2006.01)
C08L 27/06 (2006.01)      C08L 33/02 (2006.01)
C08L 75/04 (2006.01)      C08L 71/02 (2006.01)
A61F 13/53 (2006.01)      A01K 1/015 (2006.01)
B01J 20/26 (2006.01)

(52) Cooperative Patent Classification (CPC):
A01K 1/015; A61F 13/53; B01J 20/26; C08J 3/12;
C08L 23/00; C08L 27/06; C08L 33/02; C08L 71/02;
C08L 75/04

(86) International application number:
PCT/JP2021/043897

(87) International publication number:
WO 2022/124143 (16.06.2022 Gazette 2022/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 09.12.2020 JP 2020204038

(71) Applicant: Sumitomo Seika Chemicals Co., Ltd.
Kako-gun, Hyogo 675-0145 (JP)

(72) Inventors:
• SAWAKI, Hiroki
Himeji-shi, Hyogo 672-8076 (JP)
• YAMAMOTO, Tomoka
Himeji-shi, Hyogo 672-8076 (JP)

(74) Representative: Vossius & Partner
Patentanwälte Rechtsanwälte mbB
Siebertstraße 3
81675 München (DE)

(54) **WATER-ABSORBING RESIN PARTICLE, ABSORBER, AND ABSORBENT ARTICLE**

(57) Water-absorbent resin particles having: polymer particles; and a polymer disposed on at least a part of a surface of the polymer particle, in which a swelling pressure measured according to the following procedures (1) to (5) is more than 0 N/cm$^2$ and equal to or less than 1.50 N/cm$^2$.
(1) A particle group A that passes through a sieve having an opening of 400 μm but remains on a sieve having an opening of 300 μm is obtained using the water-absorbent resin particles.
(2) A tubular container having opening ends at both ends is disposed in a vertical direction.
(3) A water-absorbent resin layer is formed by disposing 0.1 g of the particle group A at a bottom in the container.
(4) 20.5 g of a weight is put on the water-absorbent resin layer in the container.
(5) A pressure of the water-absorbent resin layer with respect to the weight 60 seconds after starting to bring physiological saline into contact with the water-absorbent resin layer from a side of the bottom of the container is obtained as the swelling pressure.

## Description

### Technical Field

The present invention relates to water-absorbent resin particles, an absorber, an absorbent article, and the like.

### Background Art

Water-absorbent resin particles are widely used in various fields of sanitary materials such as diapers, hygiene products, and portable toilets; agricultural and horticultural materials such as water retention agents and soil improvement agents; and industrial materials such as waterproofing agents and condensation prevention agents. For example, an absorber containing water-absorbent resin particles has been used in an absorbent article for absorbing a liquid (for example, urine) having water as a main component. Patent Literature 1 below discloses water-absorbent resin particles that are used for absorbers of absorbent articles such as diapers.

### Citation List

### Patent Literature

[Patent Literature 1] Japanese Unexamined Patent Publication No. H06-345819

### Summary of Invention

### Technical Problem

When an absorber is used in sanitary materials, the user may feel a sense of discomfort as the absorber absorbs water and water-absorbent resin particles expand. Therefore, the absorber is required to have an excellent touch feeling after liquid absorption.

An object of one aspect of the present invention is to provide water-absorbent resin particles capable of obtaining an absorber having an excellent touch feeling after liquid absorption. An object of another aspect of the present invention is to provide an absorber and an absorbent article using the water-absorbent resin particles.

### Solution to Problem

One aspect of the present invention relates to water-absorbent resin particles having: polymer particles; and a polymer disposed on at least a part of a surface of the polymer particle, in which a swelling pressure measured according to the following procedures (1) to (5) is more than 0 N/cm$^2$ and equal to or less than 1.50 N/cm$^2$.

(1) A particle group A that passes through a sieve having an opening of 400 $\mu$m but remains on a sieve having an opening of 300 $\mu$m is obtained using the water-absorbent resin particles.
(2) A tubular container having opening ends at both ends is disposed in a vertical direction.
(3) A water-absorbent resin layer is formed by disposing 0.1 g of the particle group A at a bottom in the container.
(4) 20.5 g of a weight is put on the water-absorbent resin layer in the container.
(5) A pressure of the water-absorbent resin layer with respect to the weight 60 seconds after starting to bring physiological saline into contact with the water-absorbent resin layer from a side of the bottom of the container is obtained as the swelling pressure.

According to such water-absorbent resin particles, an absorber having an excellent touch feeling after liquid absorption can be obtained.

Another aspect of the present invention relates to an absorber containing the above-mentioned water-absorbent resin particles.

Still another aspect of the present invention relates to an absorbent article having the above-mentioned absorber.

### Advantageous Effects of Invention

According to one aspect of the present invention, water-absorbent resin particles capable of obtaining an absorber having an excellent touch feeling after liquid absorption can be provided. According to another aspect of the present invention, an absorber and an absorbent article using the water-absorbent resin particles can be provided.

**Brief Description of Drawings**

Fig. 1 is a schematic cross-sectional view showing an example of an absorbent article.
Fig. 2 is a schematic cross-sectional view for describing a method for measuring a swelling pressure.

**Description of Embodiments**

Hereinafter, embodiments of the present invention will be described in detail. However, the present invention is not limited to the following embodiments, and can be various modified and implemented within the scope of the gist thereof.

In the present specification, "acrylic" and "methacrylic" are collectively referred to as "(meth)acrylic". Similarly, "acrylate" and "methacrylate" are also referred to as "(meth)acrylate". "(Poly)" means both of a case where there is a prefix of "poly" and a case where there is no prefix thereof. In a numerical value range described in stages in the present specification, the upper limit value or the lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in Examples. "Room temperature" means 25°C $\pm$ 2°C. For materials exemplified in the present specification, one type may be used alone, or two or more types may be used in combination. The content of each component in the composition means the total amount of a plurality of substances present in the composition in a case where the plurality of substances corresponding to each component are present in the composition, unless otherwise specified. "Physiological saline" means a 0.9% by mass sodium chloride aqueous solution.

Water-absorbent resin particles of the present embodiment have polymer particles (bodies to be coated), and a polymer disposed on at least a part of the surface of the polymer particle. In the water-absorbent resin particles of the present embodiment, a swelling pressure measured according to the following procedures (1) to (5) is more than 0 N/cm$^2$ and equal to or less than 1.50 N/cm$^2$.

(1) A particle group A that passes through a sieve having an opening of 400 $\mu$m but remains on a sieve having an opening of 300 $\mu$m is obtained using the water-absorbent resin particles.
(2) A tubular container having opening ends at both ends is disposed in a vertical direction.
(3) A water-absorbent resin layer is formed by disposing 0.1 g of the particle group A at a bottom in the container.
(4) 20.5 g of a weight is put on the water-absorbent resin layer in the container.
(5) A pressure of the aqueous resin layer with respect to the weight 60 seconds after starting to bring physiological saline into contact with the water-absorbent resin layer from a side of the bottom of the container is obtained as the swelling pressure.

According to the water-absorbent resin particles of the present embodiment, an absorber having an excellent touch feeling after liquid absorption can be obtained. It is presumed that the swelling pressure is low in a state where the polymer is disposed on the surface of the polymer particles, and therefore, the water-absorbent resin particles expand gently (with a weak force), which makes it possible to prevent a deterioration of a touch feeling after liquid absorption. However, the factors for obtaining the effect are not limited to this content.

Meanwhile, in a case where a liquid provided to an absorbent article does not sufficiently permeate the absorbent article, there may be a problem of flowing of the excess liquid on the surface of the absorbent article, resulting in its leakage to the outside of the absorbent article. Therefore, an absorbent article is required to cause a liquid to permeate it at an excellent permeation rate. In this regard, according to one aspect of the water-absorbent resin particles of the present embodiment, an absorbent article having an excellent permeation rate can be obtained.

In addition, it is required in the absorbent article that, when an absorbent member is brought into contact with a liquid supply position of the absorbent article in a state where the liquid has permeated the absorbent article, the liquid that has permeated the absorbent article is not easily absorbed by the absorbent member, that is, re-wet after liquid absorption can be prevented. On the other hand, according to one aspect of the water-absorbent resin particles of the present embodiment, an absorbent article capable of preventing re-wet after liquid absorption can be obtained. In the absorbent article capable of preventing re-wet after liquid absorption, when an absorbent member is brought into contact with a liquid supply position of the absorbent article in a state where a liquid has permeated the absorbent article, the liquid that has permeated the absorbent article is not easily absorbed by the absorbent member (in other words, the amount of re-wet is small).

Therefore, according to one aspect of the water-absorbent resin particles of the present embodiment, an absorber having an excellent touch feeling after liquid absorption can be obtained, and also, an absorbent article having an excellent permeation rate and capable of preventing re-wet after liquid absorption can be obtained.

The swelling pressure (25°C) measured by the above-mentioned procedures (1) to (5) is more than 0 N/cm$^2$ and equal to or less than 1.50 N/cm$^2$ from the viewpoint of obtaining an absorber having an excellent touch feeling after liquid absorption. The swelling pressure tends to be lowered by disposing the polymer on the surface of the polymer particles, but the swelling pressure can be sufficiently lowered by changing the water-absorbent characteristics of the polymer particles. For example, the swelling pressure can be lowered by reducing the water retention amount of the polymer particles, by reducing the gel strength at the time of swelling of the polymer particles, and the like. The gel strength at the time of swelling of the polymer particles can be adjusted by the use amount of a crosslinking agent to be used when obtaining the polymer particles, and the like.

In (1) mentioned above, the particle group A that passes through a sieve having an opening of 400 μm but remains on a sieve having an opening of 300 μm is obtained using the water-absorbent resin particles of the present embodiment. The particle group A can be obtained as a particle group that remains on a sieve having an opening of 300 μm after a particle group of the water-absorbent resin particles of the present embodiment passes through a sieve having an opening of 400 μm. The particle group of the water-absorbent resin particles of the present embodiment contains particles having a particle diameter (maximum diameter) of equal to or more than 300 μm and less than 400 μm.

In (2) mentioned above, the tubular container having opening ends at both ends is disposed in a vertical direction. The tubular container has an inner space that penetrates from one opening end to the other opening end as an inner space that can store the water-absorbent resin particles. The cross-sectional shape of the inner space may be circular, and the inner diameter of the inner space may be 20.0 mm. After disposing a mesh member (nylon mesh, 255 mesh, thickness: 80 μm) at one opening end of the container, the container is disposed in the vertical direction in a state where the one opening end is positioned on the lower side in the vertical direction.

In (3) mentioned above, the water-absorbent resin layer is formed by disposing 0.1 g of the particle group A at the bottom in the container (in the inner space of the container).

In (4) mentioned above, 20.5 g of a weight (cross-sectional shape: circular shape, diameter: 19.5 mm) is put on the water-absorbent resin layer in the container.

In (5) mentioned above, the pressure of the water-absorbent resin layer with respect to the weight 60 seconds after starting to bring physiological saline into contact with the water-absorbent resin layer from a side of the bottom of the container is obtained as the swelling pressure. A glass filter (diameter: 50 mm, thickness: 5 mm, circular shape, filter hole diameter: G1) permeated with physiological saline and a filter paper (diameter: 55 mm, circular shape) are laminated in this order on the side of the bottom of the container, and thereafter, the swelling pressure 60 seconds after starting to bring the filter paper and the water-absorbent resin layer into contact with each other is measured.

From the viewpoint of being able to suitably adjust the touch feeling of the absorber after liquid absorption, and the permeation rate and the amount of re-wet of the absorbent article, the swelling pressure may be 0.001 N/cm$^2$ or more, 0.005 N/cm$^2$ or more, 0.008 N/cm$^2$ or more, 0.01 N/cm$^2$ or more, 0.02 N/cm$^2$ or more, 0.03 N/cm$^2$ or more, 0.05 N/cm$^2$ or more, 0.06 N/cm$^2$ or more, 0.08 N/cm$^2$ or more, 0.10 N/cm$^2$ or more, 0.20 N/cm$^2$ or more, 0.30 N/cm$^2$ or more, 0.40 N/cm$^2$ or more, 0.50 N/cm$^2$ or more, 0.60 N/cm$^2$ or more, or 0.70 N/cm$^2$ or more. From the viewpoint of being able to suitably adjust the touch feeling of the absorber after liquid absorption, and the permeation rate and the amount of re-wet of the absorbent article, the swelling pressure may be 1.20 N/cm$^2$ or less, 1.00 N/cm$^2$ or less, 0.80 N/cm$^2$ or less, 0.70 N/cm$^2$ or less, 0.60 N/cm$^2$ or less, 0.50 N/cm$^2$ or less, 0.40 N/cm$^2$ or less, 0.30 N/cm$^2$ or less, 0.20 N/cm$^2$ or less, 0.10 N/cm$^2$ or less, 0.08 N/cm$^2$ or less, 0.06 N/cm$^2$ or less, 0.05 N/cm$^2$ or less, 0.03 N/cm$^2$ or less, 0.02 N/cm$^2$ or less, or 0.01 N/cm$^2$ or less. From these viewpoints, the swelling pressure may be 0.001 to 1.20 N/cm$^2$, 0.001 to 1.00 N/cm$^2$, 0.001 to 0.80 N/cm$^2$, 0.001 to 0.50 N/cm$^2$, 0.001 to 0.30 N/cm$^2$, 0.001 to 0.20 N/cm$^2$, 0.001 to 0.10 N/cm$^2$, 0.001 to 0.05 N/cm$^2$, 0.001 to 0.01 N/cm$^2$, 0.005 to 1.20 N/cm$^2$, 0.005 to 1.00 N/cm$^2$, 0.005 to 0.80 N/cm$^2$, 0.005 to 0.50 N/cm$^2$, 0.005 to 0.30 N/cm$^2$, 0.005 to 0.20 N/cm$^2$, 0.005 to 0.10 N/cm$^2$, 0.005 to 0.05 N/cm$^2$, 0.005 to 0.01 N/cm$^2$, 0.01 to 1.20 N/cm$^2$, 0.01 to 1.00 N/cm$^2$, 0.01 to 0.80 N/cm$^2$, 0.01 to 0.50 N/cm$^2$, 0.01 to 0.30 N/cm$^2$, 0.01 to 0.20 N/cm$^2$, 0.01 to 0.10 N/cm$^2$, 0.01 to 0.05 N/cm$^2$, 0.02 to 1.20 N/cm$^2$, 0.02 to 1.00 N/cm$^2$, 0.02 to 0.80 N/cm$^2$, 0.02 to 0.50 N/cm$^2$, 0.02 to 0.30 N/cm$^2$, 0.02 to 0.20 N/cm$^2$, 0.02 to 0.10 N/cm$^2$, 0.02 to 0.05 N/cm$^2$, 0.10 to 1.20 N/cm$^2$, 0.10 to 1.00 N/cm$^2$, 0.10 to 0.80 N/cm$^2$, 0.10 to 0.50 N/cm$^2$, 0.10 to 0.30 N/cm$^2$, 0.10 to 0.20 N/cm$^2$, 0.20 to 1.20 N/cm$^2$, 0.20 to 1.00 N/cm$^2$, 0.20 to 0.80 N/cm$^2$, 0.20 to 0.50 N/cm$^2$, or 0.20 to 0.30 N/cm$^2$.

From the viewpoint of being able to suitably adjust the touch feeling of the absorber after liquid absorption, and the permeation rate and the amount of re-wet of the absorbent article, the water retention amount (25°C) of the physiological saline of the water-absorbent resin particles may be in the following range. The water retention amount may be 10 g/g or more, 15 g/g or more, 20 g/g or more, 22 g/g or more, 25 g/g or more, more than 25 g/g, 29 g/g or more, 30 g/g or more, more than 30 g/g, 34 g/g or more, 35 g/g or more, more than 35 g/g, 40 g/g or more, 42 g/g or more, 44 g/g or more, 45 g/g or more, 50 g/g or more, 55 g/g or more, 60 g/g or more, 70 g/g or more, 80 g/g or more, or 87 g/g or more. The water retention amount may be 100 g/g or less, 90 g/g or less, 87 g/g or less, 85 g/g or less, 80 g/g or less, 70 g/g or less, 60 g/g or less, 55 g/g or less, 50 g/g or less, 45 g/g or less, 44 g/g or less, 42 g/g or less, 40 g/g or less, 35 g/g or less, less than 35 g/g, 34 g/g or less, 30 g/g or less, less than 30 g/g, 29 g/g or less, 25 g/g or less, less than 25 g/g, or 22 g/g or less. From these viewpoints, the water retention amount may be 10 to 100 g/g, 10 to 87 g/g, 10 to 55 g/g, 10 to 40 g/g, 10 to 30 g/g, 10 to 25 g/g, 22 to 100 g/g, 22 to 87 g/g, 22 to 55 g/g, 22 to 40 g/g, 22 to 30 g/g, 22 to 25 g/g, 25 to 100 g/g, 25 to 87 g/g, 25 to 55 g/g, 25 to 40 g/g, 25 to 30 g/g, 30 to 100 g/g, 30 to 87 g/g, or 30 to 55 g/g. The water retention amount can be measured by a method described in Examples to be described later.

The medium particle diameter (25°C) of the water-absorbent resin particles of the present embodiment may be in the following range. The medium particle diameter of the water-absorbent resin particles may be 100 μm or more, 150 μm or more, 200 μm or more, 250 μm or more, 300 μm or more, 350 μm or more, 360 μm or more, 370 μm or more, 380 μm or more, 400 μm or more, 420 μm or more, or 450 μm or more. The medium particle diameter of the water-absorbent resin particles may be 800 μm or less, 700 μm or less, 600 μm or less, 500 μm or less, 450 μm or less, 420 μm or less, 400 μm or less, less than 400 μm, 380 μm or less, 370 μm or less, or 360 μm or less. From these viewpoints, the medium particle diameter of the water-absorbent resin particles may be 100 to 800 μm, 100 to 500 μm, 200 to 500 μm, 300 to 500 μm, or 350 to 500 μm. The medium particle diameter may be a particle diameter based on mass.

The polymer particles (bodies to be coated) in the water-absorbent resin particles of the present embodiment may have water absorption properties. The shape of the polymer particles is not particularly limited, and may be substantially spherical, amorphous, granular, or the like, and may be a shape in which primary particles having these shapes are aggregated, for example. The amorphous polymer particles can be obtained by crushing the mass of the polymer with a crusher, for example.

The polymer particles may be surface-crosslinked or may not be surface-crosslinked. In the surface-crosslinked polymer particles, the crosslinking density on the surface is higher than that on the inside of the particles.

The polymer particles may contain a gel stabilizer, a metal chelating agent, a flowability improver (lubricant), or the like. These components may be disposed inside the polymer particles, or on the surface of the polymer particles, or both inside and on the surface thereof.

The polymer particles can have an ethylenically unsaturated monomer as a monomer unit (can have a structural unit derived from an ethylenically unsaturated monomer). The polymer particles can also have a monomer different from the ethylenically unsaturated monomer as a monomer unit.

The ethylenically unsaturated monomer may be a water-soluble ethylenically unsaturated monomer (for example, an ethylenically unsaturated monomer having the solubility of 1 g or more in 100 g of ion-exchanged water at 25°C). Examples of the ethylenically unsaturated monomer include (meth)acrylic acid and salts thereof, (meth)acrylic acid esters (methyl (meth)acrylate, ethyl (meth)acrylate, 2-hydroxyethyl (meth)acrylate, 2-(diethylamino)ethyl (meth)acrylate, 2-(diethylamino)propyl (meth)acrylate, and the like), (meth)acrylamide-based monomers ((meth)acrylamide, N-isopropyl(meth)acrylamide, 2-(meth)acrylamide-2-methylpropanesulfonic acid and salts thereof, N,N-dimethyl(meth)acrylamide, N-methylol(meth)acrylamide, diethylaminopropyl (meth)acrylamide, and the like), and polyethylene glycol mono(meth)acrylate. From the viewpoint of being able to suitably adjust the touch feeling of the absorber after liquid absorption, and the permeation rate and the amount of re-wet of the absorbent article, the ethylenically unsaturated monomer may include at least one (meth)acrylic acid component selected from the group consisting of (meth)acrylic acid and a salt thereof, and the polymer particles may have at least one (meth)acrylic acid component selected from the group consisting of (meth)acrylic acid and a salt thereof as a monomer unit.

The content of the monomer unit of the ethylenically unsaturated monomer may be 70 to 100 mol% based on the total amount of the monomer units constituting the polymer particles. The content of the monomer unit of the (meth)acrylic acid component (total amount of the monomer unit of the (meth)acrylic acid and a salt thereof) may be 70 to 100 mol% based on the total amount of the monomer units constituting the polymer particles.

The polymer disposed on the surface of the polymer particles can prevent the polymer particles from coming into contact with a liquid to be absorbed. The polymer disposed on the surface of the polymer particles may form a cover part (coating material) that covers at least a part of the surface of the polymer particles (bodies to be coated) (where the water-absorbent resin particles may be coated resin particles having polymer particles and a cover part). It is sufficient for the cover part to cover at least a part of the surface of the polymer particle, and the cover part covers a part or the entire part of the surface of the polymer particle. The cover part may have a one-layer structure or a multilayer structure having two or more layers.

The polymer disposed on the surface of the polymer particles may be different from the polymer constituting the polymer particles. The polymer disposed on the surface of the polymer particles may be water-soluble or may not be water-soluble (may be poorly water-soluble). The polymer may contain a water-soluble component or may contain a poorly water-soluble component. The term "water-soluble" means a solubility of 1 g or more (for example, 1 to 150 g) in 100 g of ion-exchanged water at 25°C. The term "poorly water-soluble" means a solubility of less than 1 g in 100 g of ion-exchanged water at 25°C.

The water-soluble component may include a compound having a hydrophilic group. Examples of the hydrophilic group include an anionic group, a cationic group, an amphoteric group, and a nonionic group. Examples of the anionic group include a carboxyl group, a sulfonic acid group, and a phosphoric acid group. Examples of the cationic group include an amino group, an imino group, and a quaternary ammonium group. Examples of the amphoteric group include a carbobetaine group, a sulfobetaine group, and a phosphobetaine group. Examples of the nonionic group include a hydroxyl group; an amide group; a cyclic lactam group such as a pyrrolidone group and a caprolactam group; an alkoxy group; and a (poly)oxyalkylene group such as a (poly)oxyethylene group and a (poly)oxypropylene group.

Examples of compounds having a hydroxyl group include polyvinyl alcohols. Examples of compounds having an amide group include polyacrylamide. Examples of compounds having a (poly)oxyalkylene group include polyalkylene oxides (for example, polyethylene oxide) and polyalkylene glycols (for example, polyethylene glycol).

As the water-insoluble component, a water-insoluble organic compound, a water-insoluble inorganic compound, or the like can be used.

Examples of the water-insoluble organic compound include polyoxyalkylene alkyl ether (for example, polyoxyethylene alkyl ethers such as polyoxyethylene oleyl ether, polyoxyethylene lauryl ether, and polyoxyethylene stearyl ether); polyesters such as polyethylene terephthalate, polybutylene terephthalate, polyethylene naphthalate, and polybutylene naphthalate; polyamides such as nylon 6 and nylon 66; polyolefins such as polyethylene, polypropylene, ethylene-butene copolymers, and ethylene-propylene copolymers; polyurethanes such as ether-based polyurethanes, ester-based polyurethanes, and carbonate-based polyurethanes; polystyrene such as poly-$\alpha$-methylstyrene and syndiotactic polystyrene; bisphenol A; polycarbonates such as polyhexamethylene carbonate; poly(meth)acrylates such as trimethylolpropane tri(meth)acrylate, pentaerythritol tri(meth)acrylate, dipentaerythritol hexa(meth)acrylate; polyalkyl (meth)acrylates such as polymethyl (meth)acrylate; polyacetals such as polyoxymethylene, polyacetaldehyde, polypropionaldehyde, and polybutyraldehyde; vinyl halide polymers such as polyvinyl chloride and polyvinyl fluoride; polyvinylidene fluoride; polysiloxane; rubber components such as styrene-butadiene rubber, nitrile rubber, acrylic rubber, polybutadiene rubber, butyl rubber, chloroprene rubber, polyisoprene rubber, and polysulfide rubber.

Polyurethane is a reaction product of polyol and polyisocyanate. The polyol may be any compound as long as it has two or more hydroxyl groups, and diols, triols, and the like can be used. Examples of the polyols include polyether polyols, polyester polyols, polycarbonate polyols, polysiloxane polyols, polyisoprene polyols, polyolefin polyols, polybutadiene polyol, and hydrogenated polybutadiene polyols. The polyisocyanate may be any compound as long as it has two or more isocyanate groups, and diisocyanate, triisocyanate, and the like can be used. Examples of the polyisocyanates include aromatic isocyanates such as diphenylmethane diisocyanate, dimethyldiphenylmethane diisocyanate, tolylene diisocyanate (for example, tolylene-2,4-diisocyanate), xylylene diisocyanate, and p-phenylene diisocyanate; alicyclic isocyanates such as dicyclohexylmethane diisocyanate and isophorone diisocyanate; and aliphatic isocyanates such as hexamethylene diisocyanate.

The polymer disposed on the surface of the polymer particles may contain a reaction product of aldehydes and phenolic compounds; a reaction product of polyols and polycarboxylic acids; a reaction product of polyamines and polycarboxylic acids; a reaction product of phenolic compounds and carbonate esters; and a reaction product of phenolic compounds and carbonate chlorides. Examples of the aldehydes include aliphatic aldehydes such as formaldehyde, acetaldehyde, and propionaldehyde; and aromatic aldehydes such as benzaldehyde. Examples of the phenolic compounds include phenol, cresol, catechol, naphthol, and hydroquinone.

The polymer disposed on the surface of the polymer particles may include a polymer having an ethylenically unsaturated monomer as a monomer unit (a polymer having a structural unit derived from an ethylenically unsaturated monomer). Examples of the ethylenically unsaturated monomer include (meth)acrylic acid and salts thereof, (meth)acrylic acid esters (methyl (meth)acrylate, ethyl (meth)acrylate, 2-hydroxyethyl (meth)acrylate, 2-(diethylamino)ethyl (meth)acrylate, 2-(diethylamino)propyl (meth)acrylate, and the like), (meth)acrylamide-based monomers ((meth)acrylamide, N-isopropyl(meth)acrylamide, 2-(meth)acrylamide-2-methylpropanesulfonic acid and salts thereof, N,N-dimethyl(meth)acrylamide, N-methylol(meth)acrylamide, diethylaminopropyl (meth)acrylamide, and the like), and polyethylene glycol mono(meth)acrylate.

As the polymer disposed on the surface of the polymer particles, it is possible to use a chain polymerization reaction product such as poly(meth)acrylic acid, poly(meth)acrylamide, polyvinyl alcohol, polyalkylene oxide, and polyalkylene glycol; and a sequential polymerization reaction product such as polyurethane (urethane resin), phenolic resin (for example, a condensate of a phenolic compound and an aldehyde), polyester, polyamide, polycarbonate; or the like.

The water-insoluble organic compound may be acid-modified. The water-insoluble organic compound may be acid-modified with an acid anhydride (such as maleic acid anhydride, succinic acid anhydride, and phthalic acid anhydride), for example.

Examples of the water-insoluble inorganic compound include light anhydrous silicic acid, calcium silicate, silicon dioxide, talc, silicon oxide, and synthetic hydrotalcite.

From the viewpoint of being able to suitably adjust the touch feeling of the absorber after liquid absorption, and the permeation rate and the amount of re-wet of the absorbent article, the polymer disposed on the surface of the polymer particles may contain polyoxyalkylene alkyl ethers (such as polyoxyethylene stearyl ether), may contain polyurethane, or may contain polyvinyl chloride.

A method for producing water-absorbent resin particles of the present embodiment includes a water-absorbent resin particle production step of disposing the polymer on at least a part of the surface of the polymer particles. The water-absorbent resin particle production step may be a polymerization step of obtaining the polymer by polymerizing monomers on at least a part of the surface of the polymer particles, or may be a step of bringing the polymer obtained by preliminarily polymerizing monomers into contact with at least a part of the surface of the polymer particles. The polymerization reaction in the polymerization step may be a chain polymerization reaction, a sequential polymerization reaction, or the like.

The method for producing water-absorbent resin particles of the present embodiment may include a surface crosslinking step of surface-crosslinking the polymer particles before the water-absorbent resin particle production step. In the surface crosslinking step, the polymer particles can be surface-crosslinked by mixing the polymer particles and a surface crosslinking agent. Examples of the surface crosslinking agent include polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether.

The method for producing water-absorbent resin particles of the present embodiment may include a polymer particle production step of polymerizing monomers to obtain the polymer particles before the surface crosslinking step and the water-absorbent resin particle production step. In the polymer particle production step, the monomer can be polymerized once or multiple times.

For example, the polymer particles can be obtained by polymerizing a monomer including an ethylenically unsaturated monomer, and can also be obtained by polymerizing an ethylenically unsaturated monomer and a monomer other than an ethylenically unsaturated monomer. Examples of polymerization methods of an ethylenically unsaturated monomer include a reverse phase suspension polymerization method, an aqueous solution polymerization method, a bulk polymerization method, and a precipitation polymerization method.

When the ethylenically unsaturated monomer has an acid group, the acid group may be neutralized and then used in the polymerization reaction. The degree of neutralization in the ethylenically unsaturated monomer may be 10 to 100 mol%, 50 to 90 mol%, or 60 to 80 mol% of the acid group in the ethylenically unsaturated monomer.

An internal crosslinking agent may be used for obtaining the polymer particles. When the internal crosslinking agent is used in the polymerization of the monomer, a crosslinking density is substantially uniformly increased easily in almost all of the polymer particles. Examples of the internal crosslinking agent include polyglycidyl compounds such as (poly)ethylene glycol diglycidyl ether, (poly)propylene glycol diglycidyl ether, (poly)glycerin diglycidyl ether, (poly)glycerin triglycidyl ether, (polypropylene glycol polyglycidyl ether, and polyglycerol polyglycidyl ether; divinyl-based compounds; dialcohol-based compounds; and diacrylate-based compounds.

The method for producing water-absorbent resin particles of the present embodiment may include a step of classifying the water-absorbent resin particles by a sieve after the water-absorbent resin particle production step. This makes it possible to adjust particle size distribution.

An absorber of the present embodiment contains the water-absorbent resin particles of the present embodiment. The absorber of the present embodiment may contain a fibrous substance, and is a mixture containing the water-absorbent resin particles and the fibrous substance, for example. For example, the structure of the absorber may be a structure in which the water-absorbent resin particles and the fibrous substance are uniformly mixed, may be a structure in which the water-absorbent resin particles are sandwiched between the fibrous substances formed in the shape of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester, and polyolefin; and a mixture of these fibers. The fibrous substance may be used alone, or may be used in combination of two or more types. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more types.

Examples of the thermal bonding synthetic fibers include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesives include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsions include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. When the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, the thickness of the sheet-shaped absorber) may be 0.1 to 20 mm, or 0.3 to 15 mm.

The content of the water-absorbent resin particles in the absorber may be in the following range based on the total mass of the absorber or the total amount of the water-absorbent resin particles and the fibrous substance. The content of the water-absorbent resin particles may be 2% by mass or more, 10% by mass or more, 20% by mass or more, or 50% by mass or more from the viewpoint of easily obtaining sufficient water-absorbent characteristics. The content of the water-absorbent resin particles is 100% by mass or less, and from the viewpoint of easily obtaining sufficient water-absorbent characteristics, the content may be 80% by mass or less, 70% by mass or less, or 60% by mass or less. From these viewpoints, the content of the water-absorbent resin particles may be 2% to 100% by mass, 10% to 80% by mass, 20% to 70% by mass, or 50% to 60% by mass.

An absorbent article of the present embodiment has the absorber of the present embodiment. Examples of other constituent members of the absorbent article of the present embodiment include a core wrap that retains the shape of the absorber and prevents falloff or flow of the constituent member of the absorber; a liquid permeable sheet disposed on the outermost part at the side where a liquid to be absorbed enters; and a liquid impermeable sheet disposed on the outermost part at the opposite side to the side where the liquid to be absorbed enters. Examples of the absorbent article include diapers (for example, disposable diapers), toilet training pants, incontinence pads, sanitary materials (sanitary napkins, tampons, and the like), sweat pads, pet sheets, portal toilet members, and animal excrement treatment materials.

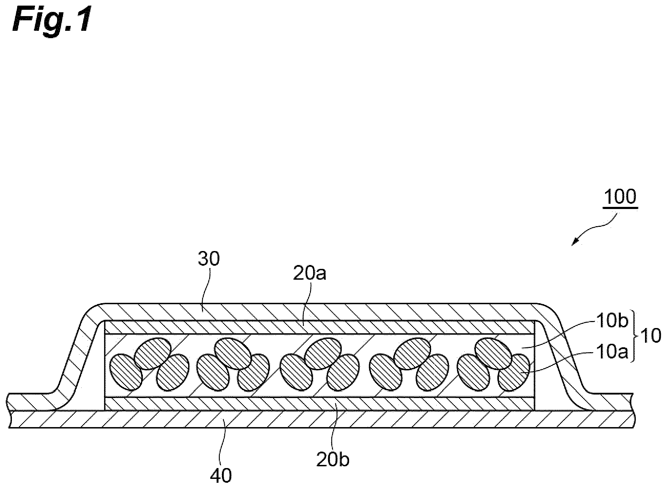

Fig. 1 is a cross-sectional view showing an example of the absorbent article. An absorbent article 100 shown in Fig. 1 has an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has water-absorbent resin particles 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in the state of being in contact with the absorber 10. The core wrap 20b is disposed on the other surface side of the absorber 10 (lower side of the absorber 10 in Fig. 1) in the state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b each have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in the state of being in contact with the core wrap 20a. Examples of the liquid permeable sheet 30 include a non-woven fabric and a porous sheet which are made of a synthetic resin such as polyethylene, polypropylene, polyester, and polyamide. The liquid impermeable sheet 40 is disposed on the outermost part at the opposite side to the liquid permeable sheet 30 in the absorbent article 100. The liquid impermeable sheet 40 is disposed on the lower side of the core wrap 20b in the state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. For example, the liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited and is appropriately adjusted according to the usage and the like of the absorbent article. In addition, a method of retaining the shape of the absorber 10 using the core wraps 20a and 20b is not particularly limited, and the absorber may be wrapped by a plurality of the core wraps as shown in Fig. 1, and the absorber may be wrapped by one core wrap.

The absorber may be adhered to a top sheet. In a case where the absorber is sandwiched between or covered by the core wraps, at least the core wrap and the top sheet may be adhered to each other, and the core wrap and the absorber may be adhered to each other while the core wrap and the top sheet are also adhered to each other. Examples of methods of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, a liquid absorbing method using the water-absorbent resin particles, the absorber, or the absorbent article of the present embodiment can be provided. The liquid absorbing method of the present embodiment includes a step of bringing a liquid to be absorbed into contact with the water-absorbent resin particles, the absorber, or the absorbent article of the present embodiment.

According to the present embodiment, a method for producing an absorber by using the water-absorbent resin particles obtained by the above-mentioned method for producing water-absorbent resin particles can be provided. The method for producing an absorber of the present embodiment includes a particle production step of obtaining the water-absorbent resin particles by the above-mentioned method for producing water-absorbent resin particles. The method for producing an absorber of the present embodiment may include a step of mixing the water-absorbent resin particles and the fibrous substance after the particle production step. According to the present embodiment, a method for producing an absorbent article using the absorber obtained by the above-mentioned method for producing an absorber can be provided. The method for producing an absorbent article of the present embodiment includes an absorber production step of obtaining the absorber by the above-mentioned method for producing an absorber. The method for producing an absorbent article of the present embodiment may include, after the absorber production step, a step of obtaining the absorbent article by using the absorber and other constituent member for the absorbent article, and in this step, for example, the absorbent article is obtained by laminating the absorber and the other constituent member for the absorbent article with each other.

**Examples**

Hereinafter, contents of the present invention will be further described using Examples and Comparative examples, but the present invention is not limited to the following Examples. In the following description, when the temperature at the time of the experimental operation is not described, the experimental operation can be performed at room temperature.

<Production of particles for evaluation>

(Example 1)

A round-bottomed cylindrical separable flask with the inner diameter of 11 cm and the internal volume of 2L equipped with a reflux cooling device, a dropping funnel, a nitrogen gas introduction tube, and a stirrer (a stirrer blade having two stages of four inclined paddle blades having the blade diameter of 5 cm) was prepared. To this separable flask, 293 g of n-heptane and 0.736 g of a maleic acid anhydride-modified ethylene-propylene copolymer (dispersant, manufactured by Mitsui Chemicals, Inc., trade name: Hi-Wax 1105A) were added to obtain a mixture. After dissolving the dispersant in n-heptane by raising the temperature to 80°C while stirring this mixture, the mixture was cooled to 55°C.

Subsequently, 92 g (acrylic acid: 1.03 mol) of an aqueous solution of 80.5% by mass acrylic acid was put to a triangular flask having the internal volume of 500 mL. Subsequently, while cooling from the outside, 103 g of an aqueous solution of 30% by mass sodium hydroxide was added dropwise to neutralize 75 mol% of acrylic acid. Thereafter, 0.092 g of hydroxyethyl cellulose (thickener, manufactured by Sumitomo Seika Chemicals Co., Ltd., trade name: HEC AW-15F), 0.092 g (0.339 mmol) of 2,2'-azobis(2-amidinopropane) dihydrochloride (azo compound), 0.0184 g (0.0681 mmol) of potassium persulfate (peroxide), 0.0202 g (0.1162 mmol) of ethylene glycol diglycidyl ether (internal crosslinking agent), and 41.0 g of ion-exchanged water were added and then dissolved to prepare a first stage monomer aqueous solution.

Then, the above-mentioned first stage monomer aqueous solution was added to the above-mentioned separable flask, and thereafter stirring was performed for 10 minutes. Thereafter, a reaction solution was obtained by adding, to the separable flask, a surfactant solution obtained by dissolving 0.736 g of sucrose stearic acid ester (surfactant, manufactured by Mitsubishi-Chemical Foods Corporation, trade name: Ryoto Sugar Ester S-370, HLB: 3) in 6.62 g of n-heptane. Then, the inside of the system was sufficiently replaced with nitrogen while stirring the reaction solution at the rotation speed of 550 rpm of the stirrer. Thereafter, the separable flask was immersed in a water bath at 70°C to raise the temperature of the reaction solution, and a first stage polymerization reaction was performed for 10 minutes to obtain a first stage polymerization slurry solution.

Subsequently, 128.8 g (acrylic acid: 1.44 mol) of an aqueous solution of 80.5% by mass acrylic acid was put to a triangular flask having the internal volume of 500 mL. Subsequently, while cooling from the outside, 143.9 g of an aqueous solution of 30% by mass sodium hydroxide was added dropwise to neutralize 75 mol% of acrylic acid. Thereafter, 0.129 g (0.475 mmol) of 2,2'-azobis(2-amidinopropane) dihydrochloride (azo compound), 0.0258 g (0.0953 mmol) of potassium persulfate (peroxide), 0.0116 g (0.0665 mmol) of ethylene glycol diglycidyl ether (internal crosslinking agent), and 15.0 g of ion-exchanged water were added and then dissolved to prepare a second stage monomer aqueous solution.

The above-mentioned first stage polymerization slurry solution was cooled to 25°C, and then the total amount of the above-mentioned second stage monomer aqueous solution was added to the first stage polymerization slurry solution to obtain a reaction solution. After sufficiently replacing the inside of the system with nitrogen, the separable flask was immersed in a water bath at 70°C again to raise the temperature of the reaction solution, and a second stage polymerization reaction was performed for 5 minutes to obtain a second stage polymerization slurry solution.

Thereafter, the temperature of the above-mentioned second stage polymerization slurry solution was raised in an oil bath at 125°C, and 241 g of water was extracted to the outside of the system while refluxing n-heptane by azeotropic distillation of n-heptane and water. Subsequently, 0.0883 g (0.5075 mmol) of ethylene glycol diglycidyl ether was added as a surface crosslinking agent, and thereafter the mixture was maintained at 83°C for 2 hours to obtain a dispersion liquid of the resin particles after surface crosslinking.

Thereafter, the temperature of the above-mentioned dispersion liquid of the resin particles after surface crosslinking was raised in an oil bath at 125°C, and drying was performed by evaporating n-heptane to obtain a dried product. This dried product was passed through a sieve having the opening of 850 $\mu$m to obtain 232.83 g of polymer particles (bodies to be coated) in the form of aggregated spherical particles.

A round-bottomed cylindrical separable flask with the inner diameter of 11 cm and the internal volume of 2 L equipped with a reflux cooling device, a nitrogen gas introduction tube, and a stirrer (a stirrer blade having two stages of four inclined paddle blades having the blade diameter of 5 cm) was prepared. To this separable flask, 250 g of n-heptane, 100 g of the above-mentioned polymer particles, and 15 g of polyoxyethylene stearyl ether (coating material, manufactured by Nihon Emulsion Co., Ltd., trade name: EMALEX 625) were added, and then stirring was performed at 1000 rpm at 85°C for 10 minutes. Thereafter, the separable flask was immersed in an oil bath at 125°C, and n-heptane was removed by evaporating it at 125°C. Then, by passing through a sieve having an opening of 850 $\mu$m, 88 g of water-absorbent resin particles (coated resin particles) having the medium particle diameter of 402 $\mu$m and having the polymer particles and the cover part (polyoxyethylene stearyl ether) covering the polymer particles was obtained as particles for evaluation.

(Example 2)

85.8 g of water-absorbent resin particles (coated resin particles) having the medium particle diameter of 371 $\mu$m and having the polymer particles and the cover part (polyoxyethylene stearyl ether) covering the polymer particles was obtained as particles for evaluation in the same manner as in Example 1 except that the use amount of ethylene glycol diglycidyl ether (internal crosslinking agent) in the first stage monomer aqueous solution was changed to 0.0607 g (0.3486 mmol); that after extracting 223 g of water to the outside of the system from the second stage polymerization slurry solution by azeotropic distillation, a dried product was obtained by raising the temperature of the reaction product in an oil bath at 125°C without performing surface crosslinking, and drying by evaporating n-heptane; and that the use amount of polyoxyethylene stearyl ether (coating material, manufactured by Nihon Emulsion Co., Ltd., trade name: EMALEX 625) was changed to 1 g.

(Example 3)

239.8 g of polymer particles (bodies to be coated) was obtained in the same manner as in Example 1 except that the use amount of ethylene glycol diglycidyl ether (internal crosslinking agent) in the first stage monomer aqueous solution was changed to 0.010 g (0.0574 mmol), that the amount of water extracted to the outside of the system from the second stage polymerization slurry solution by azeotropic distillation was changed to 239 g, and that the use amount of ethylene glycol diglycidyl ether as a surface crosslinking agent was changed to 0.7066 g (4.059 mmol).

A round-bottomed cylindrical separable flask with the inner diameter of 110 mm and the internal volume of 2 L equipped with a reflux cooling device, a dropping funnel, a nitrogen gas introduction tube, and a stirrer (a stirrer blade having two stages of four inclined paddle blades having the blade diameter of 50 mm) was prepared. 40 g of the above-mentioned polymer particles and 480 g of n-heptane were mixed in this separable flask.

8.0 g of a polyol aqueous solution obtained by mixing 0.4 g of a polyether polyol (manufactured by AGC Inc., trade name: EXCENOL 750ED) and 7.6 g of ion-exchanged water was added to the separable flask, and then stirring was performed for 30 minutes at room temperature. Thereafter, 4.78 g of an isocyanate-acetone solution obtained by mixing 0.48 g of Tolylene-2,4-diisocyanate and 4.30 g of acetone was added, and then stirring was performed for 60 minutes at room temperature to cause a sequential polymerization reaction to advance on the surface of the polymer particles, thereby obtaining a reaction product. Subsequently, the temperature of the reaction product was raised in an oil bath at 125°C to remove n-heptane by evaporating it. Then, by passing through a sieve having an opening of 850 $\mu$m, 38.2 g of water-absorbent resin particles (coated resin particles) having the medium particle diameter of 393 $\mu$m and having the polymer particles and the cover part (polyurethane) covering the polymer particles was obtained as particles for evaluation.

(Example 4)

236.8 g of polymer particles (bodies to be coated) was obtained in the same manner as in Example 1 except that 0.0736 g (0.272 mmol) of potassium persulfate (peroxide) was used instead of 2,2'-azobis(2-amidinopropane) dihydrochloride (azo compound) when preparing the first stage monomer aqueous solution, that 0.1030 g (0.3812 mmol) of potassium persulfate (peroxide) was used instead of 2,2'-azobis(2-amidinopropane) dihydrochloride (azo compound) when preparing the second stage monomer aqueous solution, that the amount of water extracted to the outside of the system from the second stage polymerization slurry solution by azeotropic distillation was changed to 245 g, and that the use amount of ethylene glycol diglycidyl ether as a surface crosslinking agent was changed to 0.0883 g (0.5075 mmol).

A coating liquid was prepared by mixing 25 g of polyvinyl chloride (manufactured by FUJIFILM Wako Pure Chemical Corporation) and 475 g of tetrahydrofuran (manufactured by FUJIFILM Wako Pure Chemical Corporation) in a beaker having the internal volume of 5 L. After 500 g of the above-mentioned polymer particles (bodies to be coated) was injected to a container of a fluid bed granulator (manufactured by POWREX CORPORATION, trade name: MP-01 mini), hot air at 40°C was blown from the lower part of the container. Subsequently, 500 g of the coating liquid was sprayed on the polymer particles wound up by hot air at 40°C. After spraying the coating liquid over 1 hour and 30 minutes, drying was performed with hot air at 40°C for 30 minutes. Thus, 506 g of water-absorbent resin particles (coated resin particles) having the medium particle diameter of 395 μm and having the polymer particles and the cover part (polyvinyl chloride) covering the polymer particles was obtained as particles for evaluation.

(Example 5)

512 g of water-absorbent resin particles (coated resin particles) having the medium particle diameter of 455 μm and having the polymer particles and the cover part (polyvinyl chloride) covering the polymer particles was obtained as particles for evaluation in the same manner as in Example 4 except that polymer particles (aqueous solution polymer, medium particle diameter: 408 μm) which were collected from a commercially available diaper sold in Japan (manufactured by Kao Corporation, trade name: Merries Pants, Dry and Soft, Air-through, S size) were used as bodies to be coated.

(Example 6)

245.37 g of water-absorbent resin particles (coated resin particles) having the medium particle diameter of 366 μm and having the polymer particles and the cover part (polyurethane) covering the polymer particles was obtained as particles for evaluation in the same manner as in Example 3 except that the amount of water extracted to the outside of the system from the second stage polymerization slurry solution by azeotropic distillation was changed to 223 g.

(Example 7)

226.47 g of water-absorbent resin particles (coated resin particles) having the medium particle diameter of 387 μm and having the polymer particles and the cover part (polyurethane) covering the polymer particles was obtained as particles for evaluation in the same manner as in Example 3 except that after extracting 256 g of water to the outside of the system from the second stage polymerization slurry solution by azeotropic distillation, a dried product was obtained by raising the temperature of the reaction product in an oil bath at 125°C without performing surface crosslinking, and drying by evaporating n-heptane.

(Comparative Example 1)

239.8 g of polymer particles having the medium particle diameter of 382 μm obtained in the same manner as in Example 3 was used as particles for evaluation.

(Comparative Example 2)

Polymer particles (aqueous solution polymer, medium particle diameter: 408 μm) which had the medium particle diameter of 363 μm and were collected from a commercially available diaper sold in Japan (manufactured by Kao Corporation, trade name: Merries S Pants, Dry and Soft, Air-through, S size) were used as particles for evaluation.

(Comparative Example 3)

510 g of coated resin particles (water-absorbent resin particles) having the medium particle diameter of 398 μm and having the polymer particles and the cover part (polyurethane) covering the polymer particles was obtained as particles for evaluation in the same manner as in Example 4 except that the coating liquid was changed to a liquid obtained by mixing 475 g of ion-exchanged water and 25 g of an emulsion (manufactured by DKS Co., Ltd., trade name: SUPERFLEX 210).

<Measurement of medium particle diameter>

The particle size distribution of 5 g of the particles for evaluation was measured using an automatic sonic sieving particle size analyzer (Robot Sifter RPS-205, manufactured by SEISHIN ENTERPRISE Co., Ltd.), JIS standard sieves having the openings of 710 $\mu$m, 600 $\mu$m, 500 $\mu$m, 425 $\mu$m, 300 $\mu$m, 250 $\mu$m, and 150 $\mu$m, and a tray. The relationship between the opening of the sieve and the integrated value of the mass percentage of the particles remaining on the sieve was plotted on a logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the medium particle diameter.

<Measurement of water retention amount>

After adding 500 g of physiological saline to a 500 mL polyethylene beaker, 2.00 g of the above-mentioned particles for evaluation was added little by little while rotating a stirring bar at a rotation speed of 600 rpm using a stirrer. After addition of the total amount of the particles for evaluation was completed, stirring was performed for 30 minutes. Subsequently, after transferring the mixture into a cotton bag, the upper part of the cotton bag was closed with a rubber band. Subsequently, centrifugation (167 G) was performed for 1 minute using a centrifuge. The mass $W_A$ was measured after dehydration. The same operation was performed without putting the particles for evaluation in the cotton bag, and the mass $W_B$ of the empty cotton bag was measured. Then, the water retention amount (25°C) of the physiological saline was calculated from the following formula. The results are shown in Table 1.

$$\text{Water retention amount } [g/g] = (W_A - W_B)/\text{mass of particles for evaluation } (= 2.00)$$

<Measurement of swelling pressure>

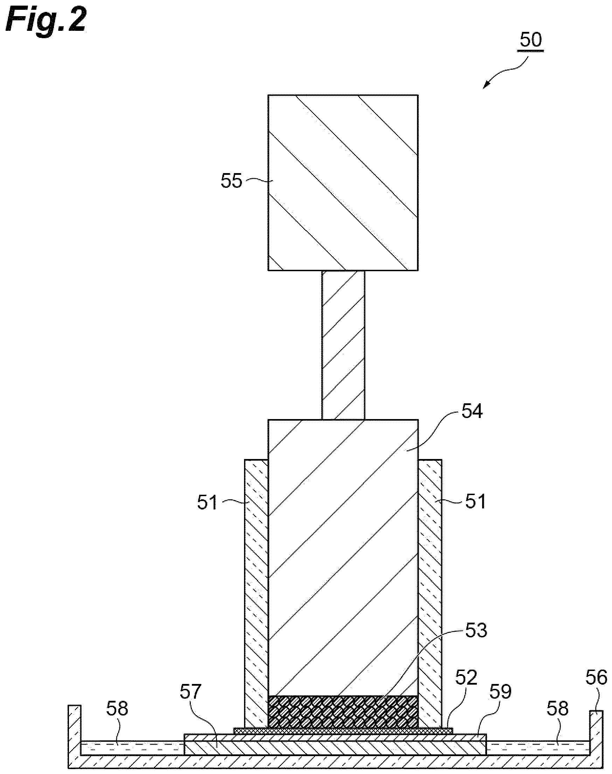

The swelling pressure (25°C) of the above-mentioned particles for evaluation was measured using a measurement device 50 shown in Fig. 2.

First, a particle group A was obtained as a particle group that remained on a sieve having an opening of 300 $\mu$m after a particle group of the particles for evaluation passed through a sieve having an opening of 400 $\mu$m.

A tubular container 51 (cylinder made of acrylic resin, opening shape: circular, inner diameter of opening: 20.0 mm, opening area: 3.14 cm$^2$, height 50 mm) having opening ends at both ends was disposed in a vertical direction. A mesh member 52 (nylon mesh, 255 mesh, thickness: 80 $\mu$m, manufactured by Nippon Tokushu fabric Inc.) was disposed at one opening end of the container 51, and thereafter, the container 51 was disposed in the vertical direction in a state where the one opening end was positioned on the lower side in the vertical direction. The mesh member 52 was disposed on the outer side of the container 51 (end surface of the container 51).

A water-absorbent resin layer 53 was formed by uniformly dispersing 0.1 g of the particle group A to dispose it at the bottom in the container 51.

20.5 g of a weight 54 (cylinder made of acrylic resin, cross-sectional shape: circular, diameter: 19.5 mm, height: 59 mm) was put on the water-absorbent resin layer 53 in the container 51. A load cell 55 of the measurement device 50 was connected to the upper part of the weight 54 in the vertical direction. As the measurement device, a small tabletop tester EZ-Test EZ-SX manufactured by Shimadzu Corporation was used.

A petri dish 56 (opening shape: circular, inner diameter: 100 mm) was horizontally placed on a measurement table of the measurement device 50. Subsequently, a glass filter 57 in a dry state (diameter: 50 mm, thickness: 5 mm, circular shape, filter hole diameter: G1) was put on the central portion of the petri dish 56, and thereafter, about 60 mL of physiological saline 58 was injected up to the position slightly below the upper surface of the glass filter 57.

A filter paper 59 (diameter: 55 mm, circular shape, JIS P 3801, No. 2, manufactured by ADVANTEC) and a liquid impermeable sheet (not shown, vinyl sheet, 50 mm × 50 mm) were put on the upper surface of the glass filter 57 permeated with the physiological saline 58, and thereafter, the physiological saline 58 was made to sufficiently permeate the filter paper 59. Subsequently, in a state where the mesh member 52 was directed downward, the container 51 containing the water-absorbent resin layer 53 was placed vertically on the liquid impermeable sheet.

The load cell 55 was lowered to bring the lower end part (pressure sensitive part) of the weight 54 close to the water-absorbent resin layer 53 and stopped when a slight pressure due to contact with the water-absorbent resin layer 53 was observed. Subsequently, the load cell 55 was moved upward little by little, and a position at which the pressure indication value reached $0 \pm 0.05$ (unit: Newton (N)) was defined as a measurement start point.

The liquid impermeable sheet on the filter paper 59 was quickly removed, and the water-absorbent resin layer 53 was brought into contact with the physiological saline 58 to start the water absorption of the water-absorbent resin layer 53. With time when the liquid impermeable sheet was removed being defined as an elapsed time t = 0 [sec], the time-dependent changes in the force generated because of the swelling of the water-absorbent resin layer 53 was measured by the load cell 55 for 60 seconds, and the value (60 seconds value) of the time at t = 60 [sec] was obtained as the swelling pressure. The results are shown in Table 1.

<Evaluation>

A swelling test was performed on absorbers produced using the particles for evaluation of Examples 1 to 7 and Comparative Examples 1 to 3. The permeation rates and re-wet were evaluated for absorbent articles produced using the absorbers of Examples 1 to 7 and Comparative Example 3.

(Production of absorber)

12.0 g of the particles for evaluation and 8.0 g of crushed pulp (manufactured by Rayonier Inc., trade name: Rayfloc) were uniformly mixed by air papermaking to produce an absorber core having the size of 40 cm $\times$ 12 cm. Subsequently, in the state of sandwiching the absorber core from its upper part and its lower part between two sheets of tissue paper (basis weight: 16 g/m$^2$) having the same size as the absorber core, the load of 141 kPa was applied to the entire absorber core for 30 seconds to press it, thereby producing an absorber (particle content: 60% by mass).

(Production of absorbent article)

The above-mentioned absorber was sandwiched by disposing a polyethylene air-through type porous liquid permeable sheet (basis weight: 22 g/m$^2$) having the same size as the absorber on the upper surface of the absorber, and disposing a polyethylene liquid impermeable sheet (basis weight: 22 g/m$^2$) having the same size as the absorber on the lower surface of the absorber, and thereby an absorbent article was obtained.

(Preparation of test solution)

9866.0 g of ion-exchanged water and a mixture of 100.0 g of sodium chloride, 3.0 g of calcium chloride dihydrate, 6.0 g magnesium chloride hexahydrate, 1.0 g of Triton X-100 (polyoxyethylene(10) octylphenyl ether, manufactured by FUJIFILM Wako Pure Chemical Corporation), and 99.0 g of ion-exchanged water were put in a container having an internal volume of 10 L, and then each of the components was completely dissolved. Subsequently, coloring with a small amount of Blue No. 1 was performed to prepare a test solution.

(Evaluation of absorber: swelling test)

A 50 mm $\times$ 50 mm square test piece was obtained from the above-mentioned absorber. The test piece was placed on the central portion of a petri dish having a diameter of 100 mm, and thereafter, 10 mL of the above-mentioned test solution was injected to the center of the test piece, and at the same time, a tester continued to touch the injection part with the finger for 60 seconds. The presence or absence of a sense of discomfort such as stimulation caused by rapid swelling, and unevenness was evaluated based on the following criteria. Five testers T1 to T5 performed evaluation and calculated a total score. A case in which the total score was 5 points or more was determined to be good. The results are shown in Table 1.

{Evaluation criteria}

2 points: no sense of discomfort at all
1 point: there is some sense of discomfort
0 points: there is a strong sense of discomfort

(Evaluation of absorbent article)

[Permeation rate]

First, the absorbent article was placed on a horizontal table. A liquid injection cylinder having an opening with the inner diameter of 3 cm was placed on the center of the absorbent article, and 80 mL of the above-mentioned test solution was injected into the cylinder at once to measure the absorption rate. The same operation was performed three times in total at intervals of 30 minutes, and the sum of the absorption rates of three times was obtained as the permeation rate [second]. The results are shown in Table 1.

[Re-wet]

After injecting the test solution three times as described above, the absorbent article was allowed to maintain its state as it was. After 1 hour from the completion of the injection of the test solution, a 10 cm square filter paper whose mass (about 75 g) had been preliminarily measured was placed near the test solution injection position on the absorbent article. Then, a weight (bottom surface: 10 cm × 10 cm, mass: 5.0 kg (about 0.7 psi)) was placed on the filter paper, and after loading for 5 minutes, the mass of the filter paper was measured, and the amount of increase in the mass of the filter paper was obtained as an amount of re-wet [g]. The results are shown in Table 1.

[Table 1]

| | Water retention amount [g/g] | Swelling pressure [N/cm$^2$] | Absorber | | | | | | Absorbent article | |
| --- | --- | --- | --- | --- | --- | --- | --- | --- | --- | --- |
| | | | Swelling test | | | | | | Permeation rate [sec] | Amount ofre-wet [g] |
| | | | T1 | T2 | T3 | T4 | T5 | Total | | |
| Example 1 | 40 | 0.29 | 2 | 1 | 1 | 2 | 2 | 8 | 98 | 33.7 |
| Example 2 | 44 | 0.06 | 2 | 2 | 1 | 2 | 2 | 9 | 103 | 40.9 |
| Example 3 | 42 | 0.02 | 2 | 2 | 1 | 2 | 1 | 8 | 116 | 44.8 |
| Example 4 | 29 | 0.01 | 2 | 2 | 2 | 2 | 2 | 10 | 125 | 41.9 |
| Example 5 | 34 | 0.79 | 1 | 2 | 1 | 2 | 2 | 8 | 140 | 38.0 |
| Example 6 | 22 | 0.16 | 1 | 2 | 0 | 2 | 1 | 6 | 83 | 58.1 |
| Example 7 | 87 | 0.03 | 1 | 2 | 2 | 2 | 2 | 9 | 276 | 14.0 |
| Comparative Example 1 | 39 | 7.06 | 1 | 0 | 0 | 0 | 1 | 2 | - | - |
| Comparative Example 2 | 31 | 3.82 | 1 | 0 | 0 | 0 | 0 | 1 | - | - |
| Comparative Example 3 | 27 | 1.91 | 0 | 1 | 0 | 0 | 1 | 2 | 112 | 58.7 |

**Reference Signs List**

10: absorber, 10a: water-absorbent resin particle, 10b: fiber layer, 20a and 20b: core wrap, 30: liquid permeable sheet, 40: liquid impermeable sheet, 50: measurement device, 51: container, 52: mesh member, 53: water-absorbent resin layer, 54: weight, 55: load cell, 56: petri dish, 57: glass filter, 58: physiological saline, 59: filter paper, 100: absorbent article.

**Claims**

1. Water-absorbent resin particles comprising:

   polymer particles; and
   a polymer disposed on at least a part of a surface of the polymer particle,

wherein a swelling pressure measured according to the following procedures (1) to (5) is more than 0 N/cm$^2$ and equal to or less than 1.50 N/cm$^2$,

(1) a particle group A that passes through a sieve having an opening of 400 μm but remains on a sieve having an opening of 300 μm is obtained using the water-absorbent resin particles,

(2) a tubular container having opening ends at both ends is disposed in a vertical direction,

(3) a water-absorbent resin layer is formed by disposing 0.1 g of the particle group A at a bottom in the container,

(4) 20.5 g of a weight is put on the water-absorbent resin layer in the container, and

(5) a pressure of the water-absorbent resin layer with respect to the weight 60 seconds after starting to bring physiological saline into contact with the water-absorbent resin layer from a side of the bottom of the container is obtained as the swelling pressure.

2. The water-absorbent resin particles according to claim 1, wherein a water retention amount of the physiological saline is 25 to 55 g/g.

3. The water-absorbent resin particles according to claim 1 or 2, wherein the polymer particle has an ethylenically unsaturated monomer as a monomer unit.

4. The water-absorbent resin particles according to claim 3, wherein the ethylenically unsaturated monomer includes at least one selected from the group consisting of (meth)acrylic acid and a salt thereof.

5. The water-absorbent resin particles according to any one of claims 1 to 4, wherein the polymer contains polyoxyalkylene alkyl ether.

6. The water-absorbent resin particles according to any one of claims 1 to 5, wherein the polymer contains polyurethane.

7. The water-absorbent resin particles according to any one of claims 1 to 6, wherein the polymer contains polyvinyl chloride.

8. An absorber comprising the water-absorbent resin particles according to any one of claims 1 to 7.

9. An absorbent article comprising the absorber according to claim 8.

# Fig.1

**_Fig.2_**

# EP 4 245 795 A1

## INTERNATIONAL SEARCH REPORT

| International application No. |
|---|
| **PCT/JP2021/043897** |

### A. CLASSIFICATION OF SUBJECT MATTER

*C08J 3/12*(2006.01)i; *C08L 23/00*(2006.01)i; *C08L 27/06*(2006.01)i; *C08L 33/02*(2006.01)i; *C08L 75/04*(2006.01)i; *C08L 71/02*(2006.01)i; *A61F 13/53*(2006.01)i; *A01K 1/015*(2006.01)i; *B01J 20/26*(2006.01)i
FI: C08J3/12 Z; C08L33/02; C08L71/02; C08L75/04; C08L27/06; C08L23/00; B01J20/26 D; A61F13/53 300; A01K1/015 A

According to International Patent Classification (IPC) or to both national classification and IPC

### B. FIELDS SEARCHED

Minimum documentation searched (classification system followed by classification symbols)

C08J3/12; C08L23/00; C08L27/06; C08L33/02; C08L75/04; C08L71/02; A61F13/53; A01K1/015; B01J20/26

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2009-96812 A (NIPPON SHOKUBAI CO LTD) 07 May 2009 (2009-05-07) entire text, all drawings | 1-9 |
| A | JP 2008-528785 A (BASF SE) 31 July 2008 (2008-07-31) entire text, all drawings | 1-9 |
| A | JP 2007-319409 A (DAIO PAPER CORP) 13 December 2007 (2007-12-13) entire text, all drawings | 1-9 |
| A | JP 3-285918 A (TOKAI RUBBER IND LTD) 17 December 1991 (1991-12-17) entire text | 1-9 |
| A | JP 2-242858 A (SANYO CHEMICAL IND LTD) 27 September 1990 (1990-09-27) entire text, all drawings | 1-9 |
| A | JP 56-159232 A (KURARAY CO) 08 December 1981 (1981-12-08) entire text | 1-9 |

☑ Further documents are listed in the continuation of Box C.　　☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **02 February 2022** | **15 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

18

**INTERNATIONAL SEARCH REPORT**

| International application No. |
| --- |
| **PCT/JP2021/043897** |

**C.      DOCUMENTS CONSIDERED TO BE RELEVANT**

| Category* | Citation of document, with indication, where appropriate, of the relevant passages | Relevant to claim No. |
| --- | --- | --- |
| A | JP 56-115259 A (KURARAY CO) 10 September 1981 (1981-09-10) <br> entire text | 1-9 |
| A | JP 57-168921 A (SUMITOMO KAGAKU KOGYO KK) 18 October 1982 (1982-10-18) <br> entire text | 1-9 |

Form PCT/ISA/210 (second sheet) (January 2015)

**INTERNATIONAL SEARCH REPORT**
Information on patent family members

International application No.

**PCT/JP2021/043897**

| Patent document cited in search report | | | Publication date (day/month/year) | Patent family member(s) | | | Publication date (day/month/year) |
|---|---|---|---|---|---|---|---|
| JP | 2009-96812 | A | 07 May 2009 | (Family: none) | | | |
| JP | 2008-528785 | A | 31 July 2008 | US | 2008/0124551 | A1 | |
| | | | | WO | 2006/082241 | A2 | |
| | | | | EP | 1846047 | A2 | |
| | | | | CN | 101115510 | A | |
| | | | | TW | 200642701 | A | |
| JP | 2007-319409 | A | 13 December 2007 | (Family: none) | | | |
| JP | 3-285918 | A | 17 December 1991 | (Family: none) | | | |
| JP | 2-242858 | A | 27 September 1990 | (Family: none) | | | |
| JP | 56-159232 | A | 08 December 1981 | (Family: none) | | | |
| JP | 56-115259 | A | 10 September 1981 | (Family: none) | | | |
| JP | 57-168921 | A | 18 October 1982 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06345819 A